# EUROPEAN PATENT APPLICATION

(11) **EP 3 372 197 A1**
(43) Date of publication of application: **12.09.2018**
(21) Application number: 17159559.8
(22) Date of filing: 07.03.2017
(51) Int. Cl.: A61F 2/16

(54) **INTRAOCULAR LENS INJECTOR AND METHOD FOR OPERATING AN INTRAOCULAR LENS INJECTOR**

(71) Applicant: Atttinger Technik AG, 8260 Stein am Rhein (CH)
(72) Inventor: ATTINGER, Jürg, 8260 Stein am Rhein (CH)
(74) Representative: Dr. Graf & Partner AG

(57) **Abstract**

An intraocular lens injector (1) for introducing an intraocular lens (100) into the eye, comprising:
- an injector body (2);
- a nozzle portion (5) comprising a distal nozzle tube (5a), the nozzle portion (5) being connected with the injector body (2);
- the nozzle portion (5) and/or the injector body (2) having a cavity (3);
- a lens holding platform (4) in the cavity (3) for accommodating the intraocular lens (100);
- the injector body (2), the cavity (3) and the nozzle portion (5) having a channel (6) extending in axial direction (P);
- an axially movable plunger (7) arranged in the channel (6) for pushing the intraocular lens (100) in axial direction (P) out of the lens holding platform (4) and into nozzle portion (5) and the distal nozzle tube (5a);
wherein the lens holding platform (4) comprises a holder (4g) adapted for preventing movement of the intraocular lens (100) at least in axial direction (P) prior to use,
wherein the holder (4g) and the plunger (7) are adapted to remove the holder (4g) when the plunger (7) is moved in direction to the distal nozzle tube (5a), so that the intraocular lens (100) can be moved in axial direction (P).

## Description

The field of invention relates to an intraocular lens injector. The field of invention further relates to a method for operating an intraocular lens injector.

### Background of the invention

With recent advances in intraocular lens (IOL) technology, cataract surgery has transitioned from being solely a treatment for visual rehabilitation to also being a refractive procedure with the aim of improving visual function and ultimately the patient's quality of life. The performance of new IOL designs is highly dependent on the handling of the IOL and the positioning of the IOL in the optical system of the eye. Depending on the type of correction, even little decentration and tilt of an IOL in the eye may decrease visual quality. Foldable IOLs made from silicone, soft acrylics and hydrogels have become increasingly popular because of the ability to fold or roll such soft lenses and insert them through a small incision.

Document EP1481652A1 discloses an intraocular lens (IOL) injector that folds the lens and provides a relatively small diameter lumen through which the lens may be pushed into the eye. The IOL injector comprises a cartridge wherein the IOL is preloaded prior to shipping. The use of this IOL injector is quite convenient, because the injector is ready to use on arrival, with no need of additional handling of the lens. However, this known IOL injector still has some drawbacks in the handling and use of the injector. For example different steps are necessary to introduce the IOL into the eye and both hands are needed to handle the IOL, and therefore errors regarding handling the IOL injector or errors regarding positioning of the IOL might occur during insertion. The handling of the IOL injector and the insertion of the IOL into the eye should be as easy and reliable as possible.

### Technical Problem to be solved

The objective of the present invention is thus to provide an IOL injector that is easy, versatile and reliable to handle.

It is also an objective of the present invention to provide an advantageous and reliable method for operating the IOL injector.

### Summary of the Invention

The above-identified objectives are solved by an IOL injector comprising the features of claim 1 and more particular by an injector comprising the features of claims 2 to 10. The objectives are further solved by a method for operating the IOL injector comprising the features of claim 11 and more particular by a method comprising the features of claims 12 to 14.

The objective is in particular solved by an intraocular lens injector for introducing an intraocular lens into the eye, comprising an injector body; a nozzle portion comprising a distal nozzle tube, the nozzle portion being connected with the injector body; the nozzle portion and/or the injector body having a cavity; a lens holding platform in the cavity for accommodating the intraocular lens; the injector body, the cavity and the nozzle portion having a channel extending in axial direction; an axially movable plunger arranged in the channel for pushing the intraocular lens in axial direction out of the lens holding platform and into nozzle portion and the distal nozzle tube; wherein the lens holding platform comprises a holder adapted for preventing movement of the intraocular lens at least in axial direction prior to use, and wherein the holder and the plunger are adapted to remove the holder when the plunger is moved in direction to the distal nozzle tube, so that the intraocular lens can be moved in axial direction. The objective is further in particular solved by a method for operating an intraocular lens injector comprising an injector body, a nozzle portion comprising a distal nozzle tube, the nozzle portion and/or the injector body having a cavity, a lens holding platform in the cavity for accommodating an intraocular lens, a holder adapted for preventing movement of the intraocular lens, and an axially movable plunger, the method comprising the steps of:
- moving the plunger in distal direction so that the plunger acts onto the holder,
- removing the holder with the plunger to thereby releasing the intraocular lens,
- moving the intraocular lens with the plunger to the nozzle portion,
- folding the intraocular lens in the nozzle portion, and
- releasing the intraocular lens through the distal nozzle tube..

The present invention improves upon prior art by providing an IOL injector having a plunger, an injector body and a nozzle portion, wherein the injector body and/or the nozzle portion form a cavity or a hollow body with a lens holding platform within the cavity. The intraocular lens is arranged in the lens holding platform. The lens holding platform comprises a holder that holds the intraocular lens stationary on the platform at least in direction of a longitudinal axis of the injector. The nozzle portion comprises a distal nozzle tube or tip with a bore, the bore communicating with the lens holding platform. The holder and the plunger are adapted such that, when moving the plunger in distal direction of the longitudinal axis, the holder is at least partially removed, so that the intraocular lens in the lens holding platform is released, so that the plunger may push the intraocular lens out of the lens holding platform to within the nozzle portion and through the distal nozzle tube to out of the tip and to within the eye. The nozzle portion preferably has a rounded entrance channel, which causes the edges of the intraocular lens between the lens haptics to fold upwardly as the lens is pushed down the bore from the lens holding platform by the plunger. In a preferred embodiment the central portion of the optic of the intraocular lens is prevented from moving upward during folding by the lens holding platform and/or the plunger. Most preferably the plunger comprises a folding mechanism that prevents the lens from moving upward during folding. In a preferred embodiment the injector body and the nozzle portion are two separate parts, and the distal end of the injector body being connected with the proximal end of the nozzle portion. In a further advantageous embodiment the injector body and the nozzle portion consist of one single part, manufactured for example by 3D-printing, so that the injector body and the nozzle portion are connected such that they form one single part. The cavity may be arranged in the nozzle portion only or in the injector body only. The cavity may also be partly arranged in the nozzle portion and partly in the injector body, such that the injector body, connected with the nozzle portion, forms the entire cavity.

In a preferred embodiment the injector comprises a lid, most preferably a hinged lid. The lid can be arranged above the lens holding platform and can be arranged to cover the cavity and the lens holding platform therein. The lens holding platform is therefore arranged beneath the lid. The lens holding platform may also be part of the lid if the lid is arranged on the bottom of the IOL injector 1. In such an embodiment the lens is hold in the part of the lid forming the lens holding platform. Opening or removing the lid means that the lens is also removed from the cavity. In a preferred embodiment the IOL injector is preloaded, which means the IOL injector is shipped with the intraocular lens within the cavity. In a preferred embodiment the intraocular lens is inserted on the manufacturing site to within the cavity and the lid is closed, and preferably also sealed, so that it cannot be opened any more during use of the IOL injector. In a further advantageous embodiment the intraocular lens is shipped separately from the IOL injector in an IOL shipping container. To load the IOL injector the lid is opened, the intraocular lens is inserted into the lens holding platform, and the lid is preferably closed again.

The IOL injector according to the invention has the advantage that the holder prevents the intraocular lens from moving during shipment and storage, in particular from moving down the nozzle portion. In a preloaded system, where the intraocular lens is already inserted in the IOL injector on the manufacturing site, the invention prevents movement during shipment, storage and during preparation of the surgical intervention. In a system where the intraocular lens is inserted into the IOL injector shortly prior to use, the invention prevents moving of the intraocular lens after insertion and during preparation of the surgical intervention. Most important, a movement of the lens is prevented during injection of a viscoelastic material into the cartridge, shortly before the intraocular lens is inserted into the eye.

The IOL injector according to the invention has the advantage that the IOL injector may be activated and the intraocular lens may be released from the lens holding platform just by pushing the plunger in distal direction. The present invention therefore provides a lens delivery system suitable for the storage, shipment and delivery of a lens into an eye without the need of handling any additional devices such as for example a device that releases the IOL within the cartridge. The IOL injector according to the invention has the advantage that it can be handled, activated and the intraocular lens inserted into the eye by using one hand only, so that the second hand is free to handle other tasks.

In a preferred embodiment the IOL injector comprises a blocking mechanism that prevents the plunger from moving during shipment, storage and preparation of the surgical intervention. Most preferably the blocking mechanism of the IOL injector is deactivated short before the intraocular lens is inserted into the eye.

In a preferred embodiment the IOL injector comprises a stopping mechanism that limits the moving distance of the plunger in distal direction. Most preferably the stopping mechanism causes increasing resistance toward the end of the moving distance, which creates a tactile feedback to the finger pushing the plunger, to provide a clear feedback that the intraocular lens is close to the exit end of the nozzle portion, or that the intraocular lens exited the distal nozzle tube. The counterforce produced by the intraocular lens passing the nozzle portion first increases, and sharply decreases as soon as the intraocular lens exits the distal nozzle tube, which usually has the effect that the whole IOL injector is pushed in distal direction. One advantage of the stopping mechanism according to the invention is that this forward movement, which occurs after the intraocular lens left the injector, can be reduced or avoided.

Other objects, features and advantages of the present invention will become apparent with reference to the drawings, and the following description of the drawings and claims.

### Brief Description of the Drawings

The figures show
- Fig. 1: a perspective view of an IOL injector;
- Fig. 2: a detailed perspective view of the cavity of the IOL injector according to figure 1;
- Fig. 3: a sectional view along A-A of figure 2;
- Fig. 4: a perspective views of the plunger hitting the intraocular lens;
- Fig. 5: a sectional view of the cavity of a further embodiment of an IOL injector;
- Fig. 6: a sectional view of the IOL injector of figure 5 with the plunger being more advanced in longitudinal direction;
- Fig. 7: a sectional view of a finger grip of the injector body and a pushing plunger;
- Fig. 8: a more detailed view of the finger grip of figure 7;
- Fig. 9: a sectional view of a further embodiment of a finger grip, a pushing plunger and an injector body;
- Fig. 10: a sectional view of the embodiment according to figure 9 with the pushing plunger being more advanced in longitudinal direction:
- Fig. 11: a more detailed view of a further injector body showing another break mechanism in detail;
- Fig. 12: a longitudinal sectional view of a further embodiment of an IOL injector;
- Fig. 13: a more detailed sectional view of the IOL injector according to figure 12;
- Fig. 14: a sectional view along B-B of figure 13;
- Fig. 15: a longitudinal sectional view of an embodiment of the nozzle portion;
- Fig. 16: a sectional view along C-C of figure 15;
- Fig. 17: a perspective views of an embodiment using a single plunger;
- Fig. 18: a sectional view of the cavity of a further embodiment of an IOL injector;
- Fig. 19: a perspective view of a further embodiment of an IOL injector.

### Description of preferred Embodiments

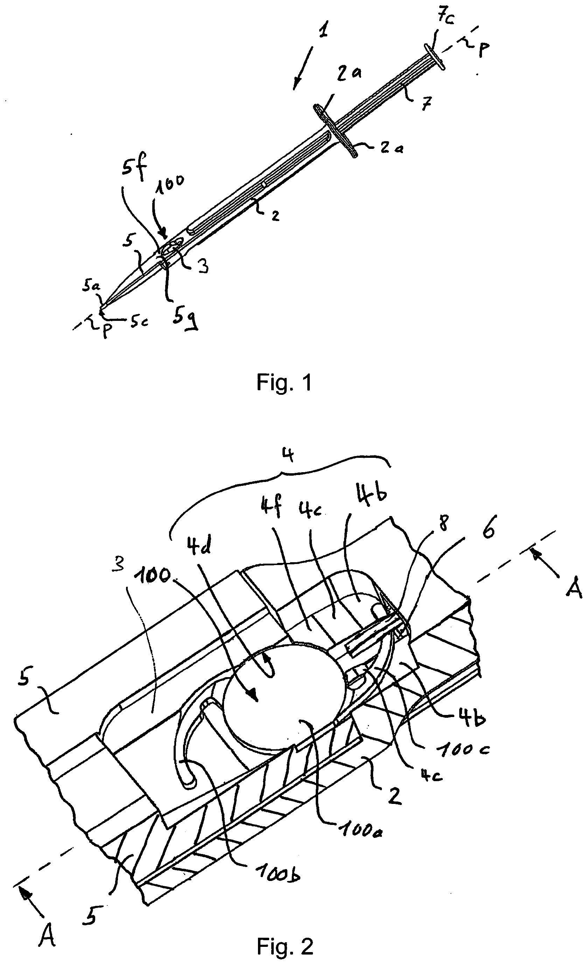

Figure 1 discloses a first embodiment of the intraocular lens (IOL) injector 1 of the present invention that generally includes an injector body 2, a nozzle portion 5 and a plunger 7. Part of the nozzle portion 5, part of the injector body 2 or part of the nozzle portion 5 and the injector body 2 together form a cavity 3 which contains a lens holding platform 4 for holding the intraocular lens (IOL) 100. The injector body 2, the cavity 3 and the nozzle portion 5 having a channel 6 extending in axial direction P. The axially movable plunger 7 is arranged in the channel 6 for pushing the intraocular lens 100 in axial direction P out of the lens holding platform 4 and into the nozzle portion 5. The nozzle portion 5 has a distal nozzle tube 5a, through which the intraocular lens 100 is pushed by moving the plunger 7 in direction of the longitudinal axis P, so that the intraocular lens 100 leaves the nozzle tube 5a at the distal end 5c. Most preferably the intraocular lens injector 1 is held by two fingers on the two finger grips 2a and by the thumb on a thumb plate 7c of the plunger 7, so that the intraocular lens injector 1 can safely be held using one hand only. One advantage of the present invention is that the intraocular lens injector 1 can also be activated in this position, so that the intraocular lens 100 may be released in the cavity using one hand only.

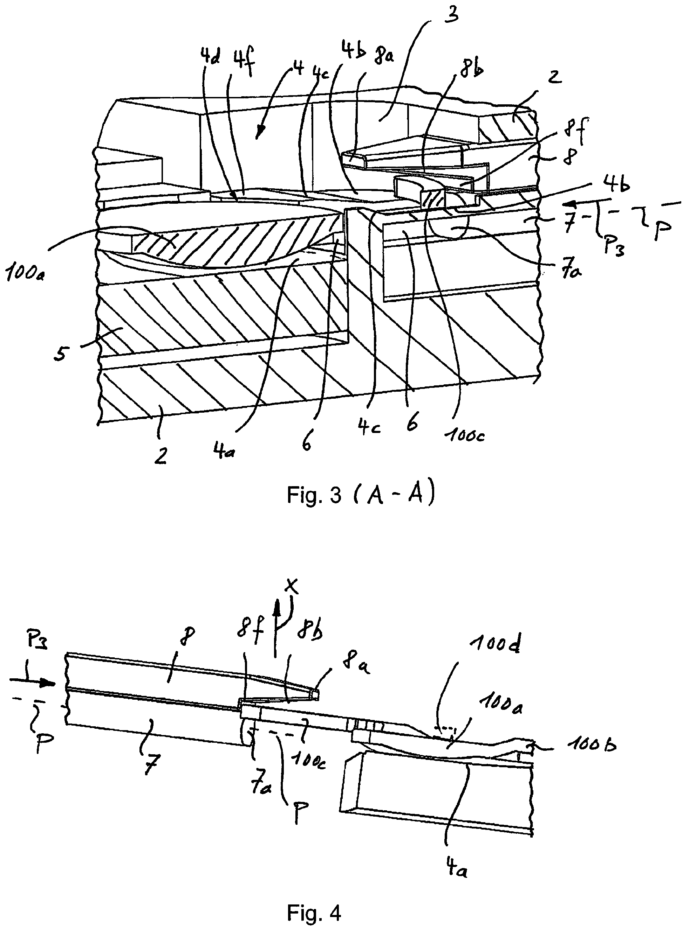

Figures 2 and 3 show sectional views of the lens holding platform 4 disclosed in figure 1 in more detail. A proximal part of the nozzle portion 5 and a distal part of the injector body 2 form together the cavity 3. The intraocular lens 100 comprises an optic part 100a, a leading haptic 100b and a trailing haptic 100c. The intraocular lens 100 is embedded in the lens holding platform 4, which comprises a bottom 4a, an edge 4d for holding the optic part 100a, and which comprises a trailing haptic rest 4b, a ramp portion 4c and a flat plane 4f. The plunger 7 is arranged in channel 6 and, after releasing plunger 7, can be moved in pushing direction P3.

As best seen in figures 3 and 4, the plunger 7 in a preferred embodiment comprises a folding mechanism 8, which comprises a second distal tip 8a, a bevel 8b and a rear tip 8f. The plunger 7 further comprises a first distal tip 7a. When moving the plunger 7 in pushing direction P3, the rear tip 8f moves the trailing haptics 100c along the ramp portion 4c to above the optic part 100a in a position indicated with 100e, while bevel 8b limits or prevents movement of the trailing haptics 100c and the optic part 100a in direction X, which is perpendicular to bottom 4a.

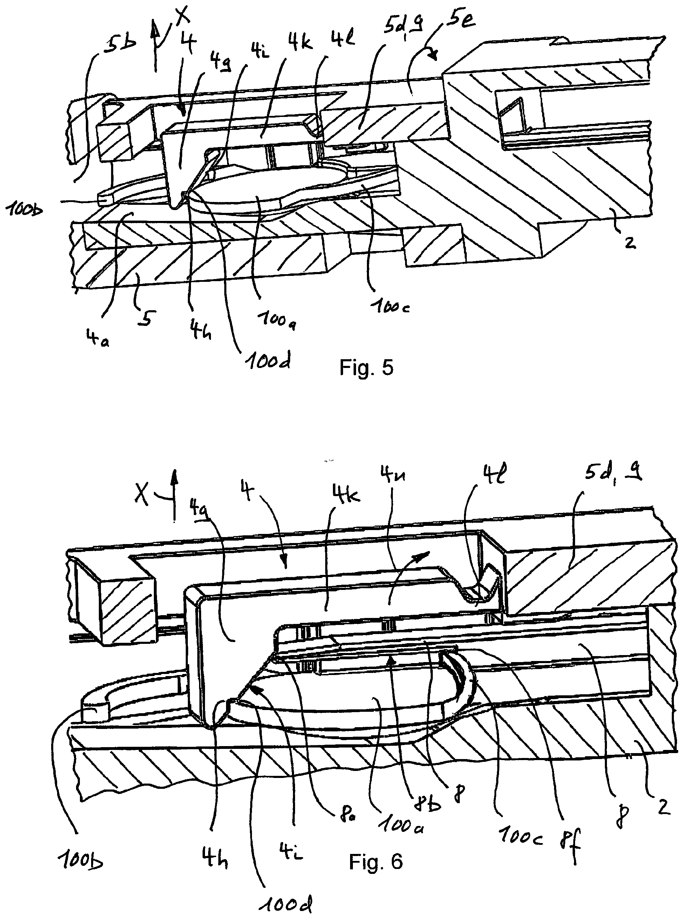

Figures 5 and 6 show a lens holding platform 4 that comprises a holder 4g adapted for preventing movement of the intraocular lens 100 at least in axial direction P prior to the use of the IOL injector 1.

The holder 4g and the folding mechanism 8 of the plunger 7 are adapted to remove the holder 4g when the plunger 7 is moved in distal direction and hits the holder 4g, so that the holder 4g is removed and the intraocular lens 100 can be moved in axial direction P into the nozzle portion 5 and the distal nozzle tube 5a. The holder 4g has a holding tip 4h adapted to hold a distal edge 100d of the intraocular lens 100 at least in axial direction P and most preferably also in direction X, perpendicular to the optic part 100a. The holder 4g comprises an engaging surface 4i arranged in channel 6 and oriented versus the first distal tip 7a of the plunger 7 or the second distal tip 8a of the folding mechanism 8 of the plunger 7, so that one of the distal tips 7a, 8a hits the engaging surface 4i when moved in direction of the distal nozzle tube 5a, as disclosed in figure 6, to remove the holder 4g. The holder 4g comprises a hinge 41 so that the holder 4g rotates in a rotating direction 4n defined by the hinge 41 when hit by the plunger 7. In the embodiment disclosed in figure 6, the plunger 7, together with its folding mechanism 8, is moved in distal direction P3. The rear tip 8f first hits the trailing haptic 100c and moves the trailing haptic 100c in distal direction, as can be seen in figure 6. The first distal tip 7a of plunger 7 then hits the optic part 100a and preferably at the same time, the second distal tip 8a hits bevel 4i, so that the holder 4g with its holding tip 4h is moved in rotating direction 4n, so that the intraocular lens 100 is released, and is moved by the first distal tip 7a of plunger 7 in distal direction P3 into the nozzle portion 5.

In a preferred embodiment the cavity 3 has a lid 5d or a cover 9. Preferably the lid 5d or the cover 9 admitting access to the cavity 3. In a preferred embodiment the hinge 41 of the holder 4g is connected to the lid 5d. In a further preferred embodiment, the lid 5d is hingedly connected to the nozzle portion 5 and/or the injector body 2. Such an embodiment allows introducing the intraocular lens 100 into the cavity 3.

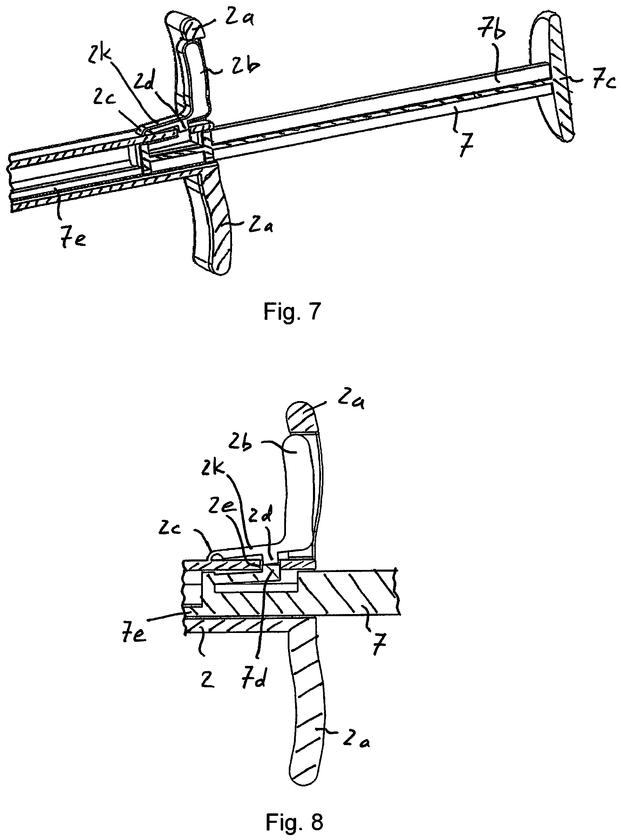

Figures 7 to 10 show a mechanism to lock and release the plunger 7 with respect to the injector body 2. The plunger 7 comprising a locking element 7d that engages with the injector body 2 to lock the plunger 7 in the injector body 2. The injector body 2 at the proximal end comprises a releasing element 2b adapted to unlock the locking element 7d, to release the plunger 7 and allow movement of the plunger 7 in axial direction P. The locking element 7d is a spring tab that engages with the injector body 2 to lock the plunger 7 in the injector body 2, wherein the injector body 2 comprising a finger grip 2a, wherein the finger grip 2a further comprising the releasing element 2b and an activation lever 2k hingedly connected to the injector body 2, whereby the activation lever 2k being able to act on the locking element 7d to release the plunger 7. For activating the intraocular lens injector 1, the intraocular lens injector 1 is held by at least one finger on the finger grips 2a, whereby the finger is acting onto the releasing element 2b, and the thumb is acting onto the thumb plate 7c of the plunger 7, so that the locking mechanism releases and the plunger 7 can be pushed in distal direction.

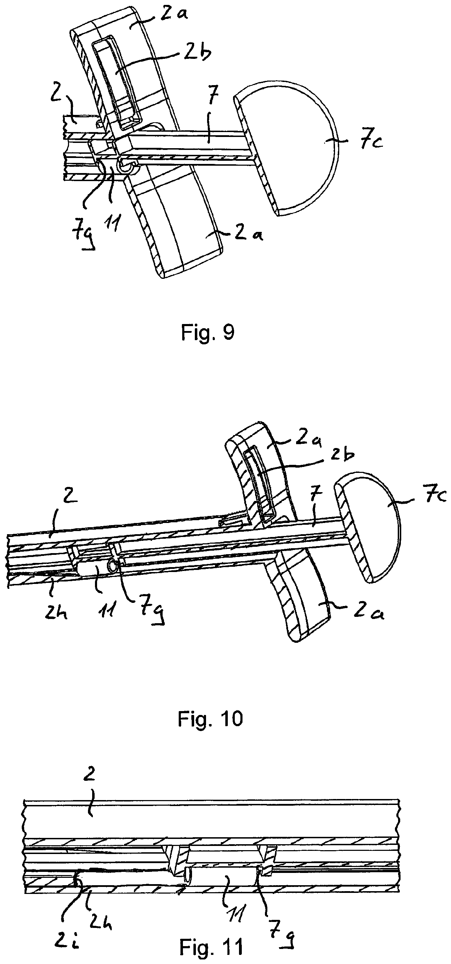

Figures 9 to 11 also show a damping element 11 acting between the plunger 7 and the injector body 2 to increase the pressure needed to move the plunger 7 in distal direction. Most preferably the pressure needed to move the plunger 7 is increased when the intraocular lens 100 arrives at the distal nozzle tube 5a. The damping element 11 is arranged such that the friction or damping force of the damping element 11 acting onto the plunger 7 moving in direction of the distal nozzle tube 5a increases up to stop movement of the plunger 7. As disclosed in figure 10, the injector body 2 may comprise a ramped portion 2h that increases friction the more the plunger 7 is pushed in distal direction. Most preferably the movement of the plunger 7 is inhibited as soon as the intraocular lens 100 exits the distal nozzle tube 5a. As disclosed in figure 11, the injector body may comprise an end stop 2i, that stops movement of the damping element 11 in distal direction. Most preferably the damping element 11 has elastic properties, so that the plunger 7 still may move a little bit in distal direction when the damping element 11 hits the end stop 2i.

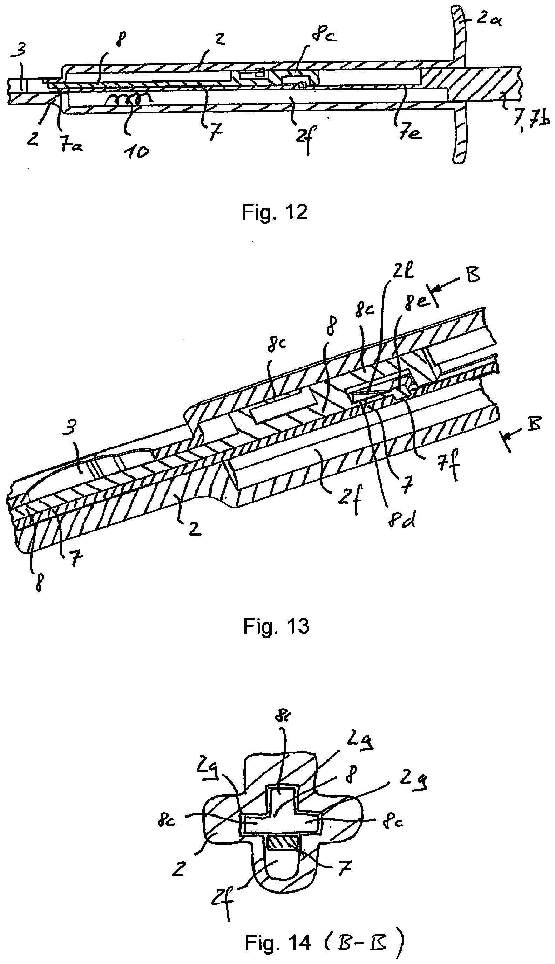

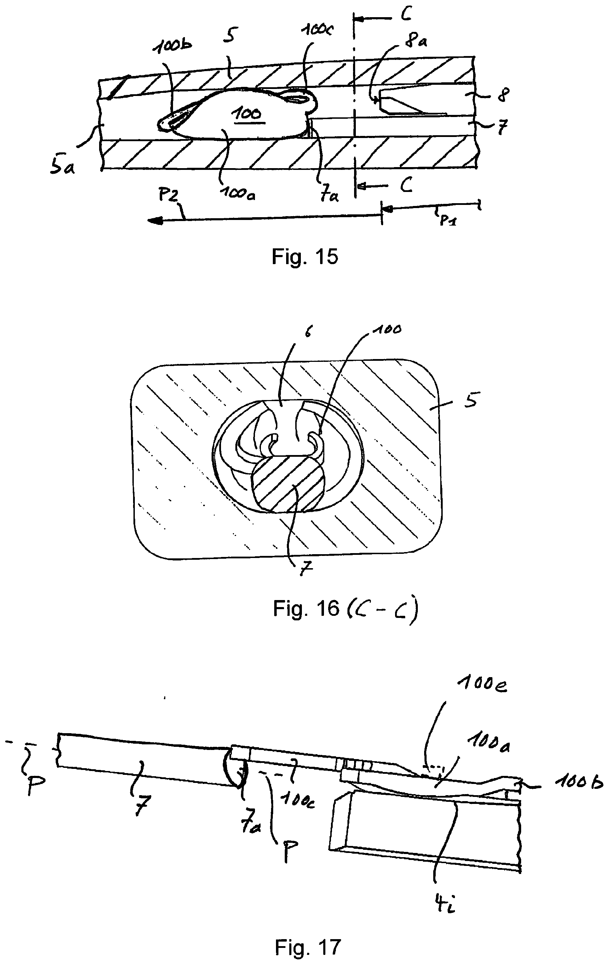

Figures 12 and 13 show longitudinal sectional views of a preferred embodiment of the injector body 2 and the plunger 7 comprising a folding mechanism 8. The plunger 7 extends from the proximal end part 7b up to the tip 7a. The folding mechanism 8 is a separate plunger arranged within channel 6 and moveable in direction of the longitudinal axis P. As disclosed in figure 13, the folding mechanism 8 comprises guiding elements 8c which contact the outer wall of the injector body 2, so that the folding mechanism 8 is guided between the outer wall and the plunger 7. Figure 14 shows a sectional view along B-B of figure 13. The folding mechanism 8 comprises three guiding elements 8c. Below the plunger 7 an optional spring chamber 2f is arranged. The plunger 7 and the folding mechanism 8 are releasably connected by connecting means 7f, 8e. The folding mechanism 8 comprises a locking element 8e and the plunger 7 comprises a recess 7f, so that the folding mechanism 8 is connected with the plunger 7, so that they move together in direction of the longitudinal axis P, up to the end of the first displacement distance P1, as disclosed in figure 15. The inner wall of the injector body 2 comprises a groove or ramp 21 which lifts the locking element 8e when the folding mechanism 8 is slidably advancing in direction of the distal end 5c and reaches the end of the first displacement distance P1, so that the folding mechanism 8 is not connected any more with the plunger 7. The movement of the folding mechanism 8 is therefore stopped, whereas the plunger 7 continues to move as long as the surgeon applies a pressure onto the thumb plate 7d. There are various options to build connecting means 7f, 8e, such as mechanical connecting means, or motor driven connecting means. In a preferred embodiment, a spring 10 is inserted in the chamber 2f, for example in the embodiment disclosed in figure 12. A spring 10 extending within the chamber 2f and acting on the distal side onto the injector body 2 and on the proximal side onto the proximal end part 7b of the plunger 7 has the effect, that the plunger 7 returns back to the position disclosed in figure 15, as soon as no pressure is applied onto the thumb plate 7d. In addition the spring gives a tactile feedback to the surgeon when pushing the thumb plate 7d.

Figure 15 shows a longitudinal sectional view of the nozzle portion 5 and figure 16 a sectional view along C-C of figure 15. During movement of the plunger 7 and its folding mechanism 8 in distal direction, the movement of the folding mechanism 8 is stopped at the end of a first displacement distance P1, and the plunger 7 continues moving in channel 6, so that the intraocular lens 100 enters the distal nozzle tube 5a and then leaves the distal nozzle tube 5a. The movement of plunger 7 is stopped at the end of the second push distance P2. Figure 16 schematically shows the intraocular lens 100 within channel 6 of the nozzle portion 5, with haptics 100b, 100c folded in axial direction, and being compacted and pushed by the plunger 7 in distal direction and afterwards through the distal nozzle tube 5a.

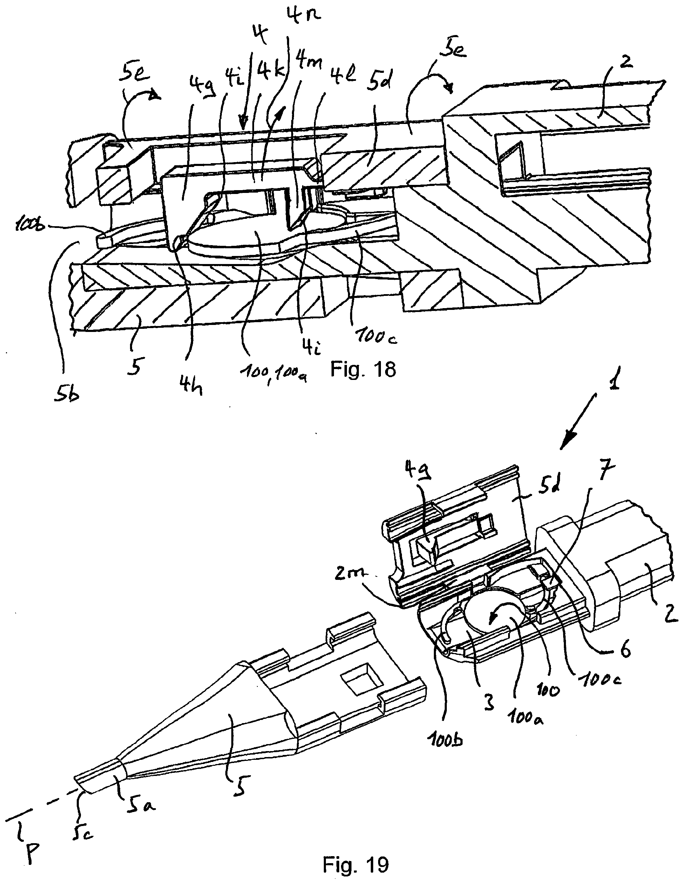

Figure 17 shows a further embodiment of a plunger 7, the plunger 7 having no additional folding mechanism 8. Figure 18 shows a sectional view of the cavity 4 of a further embodiment of an IOL injector 1. In contrast to the embodiment disclosed in figure 5, the embodiment according to figure 18 comprises an activation member 4m comprising a bevel 4i. The plunger 7 disclosed in figure 17, when moved in distal direction, first hits with its distal tip 7a the trailing haptic 100c and moves the trailing haptic in position 100e, as disclosed in figure 17. The distal tip 7a then hits the bevel 4i of the activation member 4m so that the holder arm 4k and the holder 4g move in rotating direction 4n, so that the holding tip 4h is moved. The intraocular lens 100 may then be pushed into the nozzle portion 5.

The perspective view of a further embodiment of an IOL injector 1 according to figure 19 shows a nozzle portion 5 comprising the distal nozzle tube 5a, and shows a injector body 2 having a cavity 3 and a lens holding platform 4, holding the intraocular lens 100. The plunger 7 arranged in a channel 6 is projecting to within the cavity 3. The holder 4g being connected to the lid 5d by a hinge 41, and the lid 5d being hingedly connected by a hinge 2m to the injector body 2. In a further embodiment the lid 5d may be arranged on the bottom of the cavity 3, the lid 5d preferably comprising the lens holding platform 4, so that when the lid 5d is removed, the lens holding platform 4 and the lens 100 hold therein is also removed. In such an embodiment, the upper part delimiting the cavity 3 and comprising the holder 4g may be fixedly connected with or be part of the injector body 2 and/or the nozzle tube 5a.

As disclosed in figure 1, the nozzle portion 5 may comprise an entry channel 5f and an exit channel 5g that is fluidly connected to the cavity 3. Channel 5f in combination with channel 5g allows a suitable viscoelastic material to be injected into cavity 3 and/or nozzle portion 5 without raising or removing lid 5d. The viscoelastic material assists in the expression of lens 100 out of nozzle portion 5. In a preferred embodiment the location of channels 5f, 5g assure that when the viscoelastic material is injected, the viscous nature of the material pushes leading haptic 100b proximally toward optic part 100a, thereby assisting in folding the intraocular lens 100.

## Claims

1. Intraocular lens injector (1) for introducing an intraocular lens (100) into the eye, comprising:
- an injector body (2);
- a nozzle portion (5) comprising a distal nozzle tube (5a), the nozzle portion (5) being connected with the injector body (2);
- the nozzle portion (5) and/or the injector body (2) having a cavity (3);
- a lens holding platform (4) in the cavity (3) for accommodating the intraocular lens (100);
- the injector body (2), the cavity (3) and the nozzle portion (5) having a channel (6) extending in axial direction (P);
- an axially movable plunger (7) arranged in the channel (6) for pushing the intraocular lens (100) in axial direction (P) out of the lens holding platform (4) and into nozzle portion (5) and the distal nozzle tube (5a);
**characterized in**
**that** the lens holding platform (4) comprises a holder (4g) adapted for preventing movement of the intraocular lens (100) at least in axial direction (P) prior to use,
wherein the holder (4g) and the plunger (7) are adapted to remove the holder (4g) when the plunger (7) is moved in direction to the distal nozzle tube (5a), so that the intraocular lens (100) can be moved in axial direction (P).

2. Intraocular lens injector according to claim 1, wherein the holder (4g) has a holding tip (4h) adapted to hold a distal edge (100d) of the intraocular lens (100).

3. Intraocular lens injector according to claim 2, wherein the holder (4g) comprises an engaging surface (4i) arranged in the channel (6) and oriented versus a distal tip (7a) of the plunger (7), so that the distal tip (7a) hits the engaging surface (4i) when moved in direction of the distal nozzle tube (5a), to remove the holder (4g).

4. Intraocular lens injector according to claim 3, wherein the holder (4g) comprises a hinge (41) so that the holder (4g) rotates in a rotating direction (4n) defined by the hinge (41) when hit by the plunger (7).

5. Intraocular lens injector according to one of the preceding claims, wherein the cavity (3) having a lid (5d) admitting access to the cavity (3).

6. Intraocular lens injector according to claim 5, wherein the hinge (41) of the holder (4g) is connected to the lid (5d), and wherein the lid (5d) is hingedly connected to the nozzle portion (5) and/or the injector body (2).

7. Intraocular lens injector according to one of the preceding claims, wherein the plunger (7) comprising a locking element (7d) that engages with the injector body (2) to lock the plunger (7) relative to the injector body (2), and wherein the injector body (2) at the proximal end comprises a releasing element (2b) adapted to unlock the locking element (7d), to release the plunger (7) and allow movement of the plunger (7) in axial direction (P).

8. Intraocular lens injector according to claim 7, wherein the locking element (7d) is a spring tab that engages with the injector body (2) to lock the plunger (7) in the injector body (2), wherein the injector body (2) comprising a finger grip (2a), wherein the finger grip (2a) further comprising the releasing element (2b), an activation lever hingedly connected to the injector body (2), the activation lever being able to act on the spring tab of the plunger (7) to release the plunger (7).

9. Intraocular lens injector according to one of the preceding claims, wherein a damping element (11) acting between the plunger (7) and the injector body (2) to increase the pressure needed to move the plunger (7) in direction of the distal nozzle tube (5a).

10. Intraocular lens injector according to claim 9, wherein the damping element (11) is arranged such that the friction or damping force of the damping element (11) acting onto the plunger (7) moving in direction of the distal nozzle tube (5a) increases up to stop movement of the plunger (7).

11. A method for operating an intraocular lens injector (1) comprising an injector body (2), a nozzle portion (5) comprising a distal nozzle tube (5a), the nozzle portion (5) and/or the injector body (2) having a cavity (3), a lens holding platform (4) in the cavity (3) for accommodating an intraocular lens (100), a holder (4g) adapted for preventing movement of the intraocular lens (100), and an axially movable plunger (7), the method comprising the steps of:
- moving the plunger (7) in distal direction so that the plunger (7) acts onto the holder (4g),
- removing the holder (4g) with the plunger (7) to thereby releasing the intraocular lens (100),
- moving the intraocular lens (100) with the plunger (7) to the nozzle portion (5),
- folding the intraocular lens (100) in the nozzle portion (5), and
- releasing the intraocular lens (100) through the distal nozzle tube (5a).

12. The method of claim 11, further comprising the steps of:
- holding the finger grip (2a) and thereby activating the releasing element (2b) to unlock the plunger (7) from the injector body (2), and
- moving the plunger (7) in distal direction.

13. The method of claim 12, further comprising the step of preventing movement of the plunger (7) in distal direction when the intraocular lens (100) has been released.

14. The method of claim 12 or 13, further comprising the step of increasing the pressure needed to move the plunger (7) when moving the plunger (7) in distal direction.
